(19) Europäisches Patentamt
European Patent Office
Office européen des brevets

(11) Veröffentlichungsnummer: **0 009 752**
**B1**

(12) **EUROPÄISCHE PATENTSCHRIFT**

(45) Veröffentlichungstag der Patentschrift: 08.04.81

(51) Int. Cl.³: C 07 D 319/06

(21) Anmeldenummer: 79103630.4

(22) Anmeldetag: 25.09.79

(54) **3-Chlor-3-methyl-butan- bzw. 3-Methyl-2-buten-1,4-dial-bis-acetale, ein Verfahren zur Herstellung dieser Verbindungen sowie deren Verwendung.**

(30) Priorität: 30.09.78 DE 2842715

(43) Veröffentlichungstag der Anmeldung: 16.04.80 Patentblatt 80/8

(45) Bekanntmachung des Hinweises auf die Patenterteilung: 08.04.81 Patentblatt 81/14

(84) Benannte Vertragsstaaten: CH DE FR GB NL

(56) Entgegenhaltungen: US - A - 3 671 590

(73) Patentinhaber: BASF Aktiengesellschaft
Carl-Bosch-Strasse 38
D-6700 Ludwigshafen (DE)

(72) Erfinder: Jaedicke, Hagen, Dr. Dipl.-Chem.
Budapester Strasse 49
D-6700 Ludwigshafen (DE)
Erfinder: Paust, Joachim, Dr. Dipl.-Chem.
Ringstrasse 3
D-6701 Neuhofen (DE)

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 009 752 B1

# 3-Chlor-3-methyl-butan- bzw. 3-Methyl-2-buten-1,4-dial-bis-acetale, ein Verfahren zur Herstellung dieser Verbindungen sowie deren Verwendung

Die vorliegende Erfindung betrifft Butan- bzw. Butandialbisacetale der allgemeinen Formel I

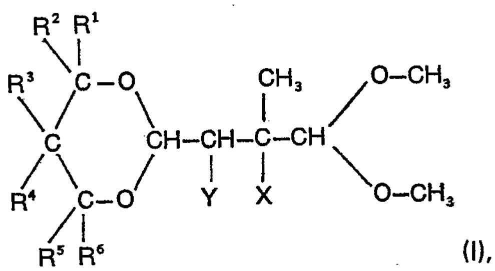

(I),

in der
$R^1$ bis $R^6$ für H, $-CH_3$ oder $-C_2H_5$, vorzugsweise H oder $-CH_3$ stehen, wobei vorzugsweise nur 1 bis 4 der Reste $R^1$ bis $R^6$ für $-CH_3$ und die restlichen für $-H$ stehen;
und
Y für H steht wenn X Cl bedeutet (Ia)
oder
Y und X zusammen für eine weitere Bindung zwischen den Y und X tragenden C-Atomen stehen (b),
sowie ein Verfahren zu deren Herstellung und deren Verwendung zur Herstellung der für Terpensynthesen begehrten 3 - Methyl - fumardialdehyd - 1 - monoacetale(trans - 3 - Methyl - 2 - buten - 1,4 - dial - 1 - acetalen).

Die trans - 3 - Methyl - 2 - buten - 1,4 - dial - 1 - monoacetale haben große Bedeutung, da es mit ihrer Hilfe möglich ist, successive Wittig-Reaktionen zu zahllosen Verbindungen mit biologischer und pharmakologischer Bedeutung durchzuführen. Beispielsweise kann man durch Umsetzen von trans - 3 - Methyl - 2 - buten - 1,4 - dial - 1 - acetalen mit dem Ylid von $\beta$ - Ionylidenäthyltriphenylphosphoniumsalzen und anschließende Hydrolyse auf einfache Weise das begehrte Retinal erhalten. Retinal besitzt die gleiche Wirksamkeit wie Vitamin A. Aus Retinal kann man außerdem durch einfache Wittig-Reaktion mit dem aus Retinol leicht erhältlichen Retinyltriphenylphosphoniumsalz auf sehr wirtschaftliche Weise $\beta$-Carotin herstellen.

Für die Herstellung der trans - 3 - Methyl - 2 - buten - 1,4 - dial - 1 - acetale sind verschiedene Verfahren bekannt.

So ist z.B. der DOS 2 225 612 ihre Herstellung durch Oxydation von cyclischen 3 - Methyl - 2 - buten - 4 - ol - 1 - al - acetalen mit schwefelsaurer Chromsäurelösung in Aceton beschrieben. Dieses Verfahren verläuft mit relativ guten Ausbeuten. Jedoch ist seine technische Realisierung durch die Verwendung von Chromsäure als Oxydationsmittel und die durch die Giftigkeit von Chromverbindungen bedingten großen Abwasserprobleme sehr erschwert.

Aus der DOS 2 357 752 ist es bekannt, die begehrten trans - 3 - Methyl - 2 - buten - 1,4 - dial - 1 - acetale durch Oxydation der entsprechenden 3 - Methyl - 2 - buten - 1 - al - acetale mit Selenoxid in bestimmten Lösungsmitteln herzustellen. Bei diesem Verfahren sind die erzielten Ausbeuten unbefriedigend. Außerdem ist die technische Durchführung des Verfahrens durch die große Toxizität des Selens sehr problematisch.

Weiterhin ist aus der DOS 2 513 999 bekannt, die trans - 3 - Methyl - 2 - buten - 1,4 - dial - 1 - acetale ausgehend von Crotonaldehydacetalen durch Ozonolyse mit anschließender reduktiver Aufarbeitung, Grignard-Vinylierung und Acetylierung der erhaltenden Glyoxalmonoacetale sowie Hydroformylierung der erhaltenen neuen 2 - Acetoxy - but - 3 - en - 1 - al - acetale und Eliminierung von Essigsäure herzustellen. Jedoch bringen die zur Durchführung dieses Verfahrens notwendigen vielen und teilweise aufwendigen Verfahrensschritte relativ hohe Herstellkosten mit sich.

Es war daher die Aufgabe der Erfindung ein Verfahren zu entwickeln, das es ermöglicht, die begehrten trans - 3 - Methyl - 2 - buten - 1,4 - dial - 1 - acetale auf technisch besonders einfache Weise, d.h. mit möglichst geringem Arbeitsaufwand herzustellen.

Mit den 3 - Chlor - 3 - methyl - butan - 1,4 - dial - bisacetalen (Ia) bzw. 3 - Methyl - 2 - buten - 1,4 - dial - bisacetalen (Ib) wurden neue Verbindungen gefunden, über die die begehrten trans - 3 - Methyl - 2 - buten - 1,4 - dial - 1 - acetale technisch besonders vorteilhaft erhalten werden können, da sie einerseits technisch auf sehr einfache und billige Weise aus gut zugänglichen Ausgangsstoffen hergestellt werden können und andererseits auf sehr einfache Weise in die trans - 3 - Methyl - 2 - buten - 1,4 - dial - 1 - acetale überführt werden können.

Die neuen Chlorbutan- bzw. Butendialbisacetale der allgemeinen Formel I können dadurch hergestellt werden, daß man.

A Ein Sechsringacetal von 3 - Methyl - 3 - buten - 1 - al der allgemeinen Formel II

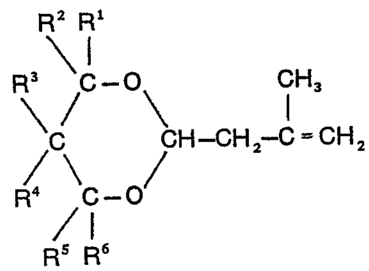

(II),

in Gegenwart von Methanol, Salzsäure und gegebenenfalls einem unter den Reaktionsbedingungen inerten Lösungsmittel mit Nitrosylchlorid oder einem Salpetrigsäureester der

allgemeinen Formel III

$$R^7—O—N{=}O \qquad (III)$$

in der $R^7$ für Alkyl mit 1 bis 6 C-Atomen, insbesondere für Methyl, Äthyl oder Isopropyl steht, zu Verbindungen der Formel I, in der Y für —H und X für Cl steht (Ia) umsetzt und

B gewünschtenfalls aus diesen in an sich bekannter Weise Chlorwasserstoff eliminiert.

Es war sehr überraschend, daß bei der Umsetzung der olefinisch ungesättigten Acetale der Formel II mit Nitrosylchlorid oder Alkylnitriten in Gegenwart von Methanol und H Cl die neuen 2 - Chlor - 2 - methyl - butan - 1,4 - dial - bisacetale in Ausbeuten bis zu 80% (bei einem Umsatz von etwa 50%) erhalten werden können, da aus der US—PS 3 671 590 bekannt war, daß bei der Umsetzung von Olefinen mit organischen Nitriten in Gegenwart von sauren Katalysatoren und ggf. in Gegenwart von Lösungsmitteln, wie u.a. Methanol, bei Temperaturen von —30 bis etwa +250°C im allgemeinen eine der Spaltung durch Ozon ähnliche Spaltung der Doppelbindung des Olefins auftritt, und daher zu erwarten war, daß unter den erfindungsgemäßen Reaktionsbedingungen eine Spaltung der olefinischen Doppelbindung der Acetale der Formel II erfolgen würde.

Gegenstand der Erfindung ist weiterhin die Verwendung der neuen Chlorbutan-bzw. Butendialbis-acetale der allgemeinen Formel I zur Herstellung von 3 - Methyl - fumardialdehyd - 1 - monoacetalen der allgemeinen Formel IV

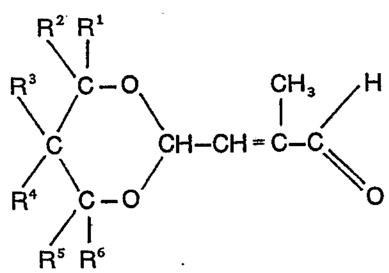

(IV),

in der $R^1$ bis $R^6$ die oben angegebene Bedeutung haben, durch Einwirken verdünnter wäßriger Säuren auf die neuen Butendialbisacetale der allgemeinen Formel Ib.

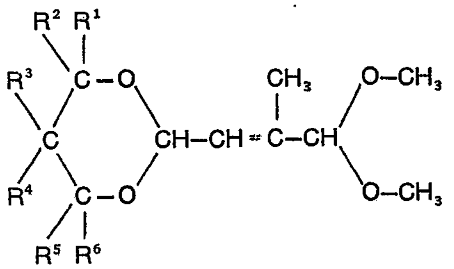

(Ib)

Gegenstand der Erfindung ist außerdem ein Verfahren zur Herstellung von 3 - Methyl - fumardialdehyd - 1 - monoacetalen der allgemeinen Formel IV

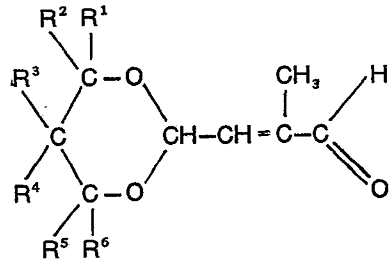

(IV),

in der $R^1$ bis $R^6$ die oben angegebene Bedeutung haben, das dadurch gekennzeichnet ist, daß man

A ein Sechsringacetal von 3 - Methyl - 3 - buten - 1 - al der allgemeinen Formel II

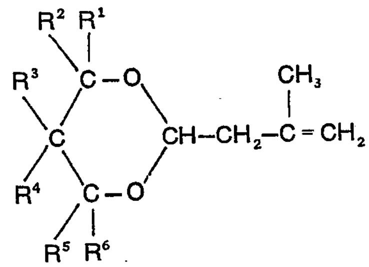

(II),

in Gegenwart von Methanol, Salzsäure und gegebenenfalls einem unter den Reaktionsbedingungen inerten Lösungsmittel mit Nitrosylchlorid oder einem Salpetrigsäureester der allgemeinen Formel III

$$R^7—O—N{=}O \qquad (III)$$ in der

$R^7$ für Alkyl mit 1 bis 6 C-Atomen, insbesondere Methyl, Äthyl, Alkyl oder Isopropyl steht, umsetzt,

B aus den so erhaltenen neuen Chlorbutandialbisacetalen der allgemeinen Formel Ia

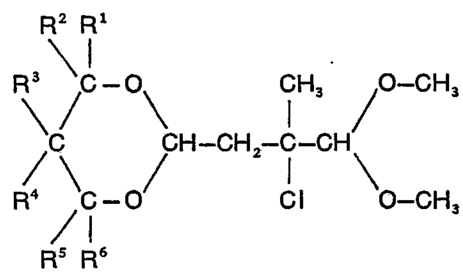

(Ia),

in der $R^1$ bis $R^6$ die oben angegebene Bedeutung haben, in an sich bekannter Weise Chlorwasserstoff eliminiert und

C die dabei erhaltenen Butendialbisacetale (2 - Methyl - fumardialdehydbisacetale) der allgemeinen Formel Ib

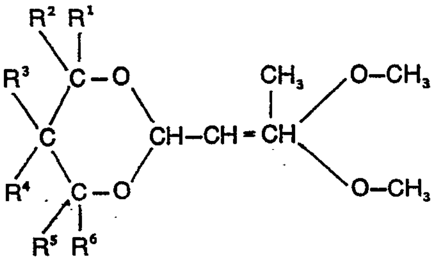

(Ib)

durch Einwirken verdünnter wäßriger Säuren partiell verseift.

Die als Ausgangsstoffe II notwendigen Sechsringacetale von 3 - Methyl - 3 - buten - 1 - al können auf einfache Weise durch Acetalisieren aus 3-methyl-3-buten-1-al erhalten werden, welches durch oxidierendes Dehydrieren von 3 - Methyl - 3 - buten - 1 - ol in Gegenwart von Mischkatalysatoren z.B. von Silber und/oder Kupfer und Metalloxiden (vgl. DE—PS 2 243 810) hergestellt werden kann. Das 3 - Methyl - 3 - buten - 1 - ol seinerseits ist leicht durch Umsetzen von Isobutylen mit Formaldehyd herstellbar.

Als geeignete Ausgangsstoffe II seien genannt die Acetale von 3 - Methyl - 3 - buten - 1 - al mit den Diolen Propan - 1,3 - diol; Butan - 1,3 - diol; Pentan - 2,4 - diol, 2,2 - Dimethyl - propan - 1,3 - diol und 2 - Methyl - propan - 1,3 - diol. Als besonders geeignet heben wir hervor die Acetale von 3 - Methyl - 3 - buten - 1 - al mit 2,2 - Dimethyl - propan - 1,3 - diol und 2 - Methyl - propan - 1,3 - diol das 3 - Methyl - 3 - buten - 1 - al- (2′,2′ - dimethyl - propylen - 1,3) -acetal und das 3 - Methyl - 3 - buten - 1 - al - (2′ - methyl - propylen - 1,3) - acetal.

Die Umsetzung der Ausgangsverbindungen II zu den Chlorbutan - dialbisacetalen kann mit Nitrosylchlorid oder mit einem Salpetrisäureester erfolgen. Besonders vorteilhaft gestaltet sich die Umsetzung bei Verwendung von Methylnitrit.

Der Chlorwasserstoff wird im allgemeinen als gasförmiges in Methanol gelöstes H Cl vorgelegt oder als gasförmiges H Cl in das Reaktionsgemisch eingeleitet. Bei Verwendung von konzentrierten wäßrigen H Cl-Lösungen werden wesentlich schlechters Ausbeuten erzielt.

Die Molverhältnisse, in denen die Reaktionspartner eingesetzt werden, können innerhalb weiter Grenzen schwanken, Man verwendet im allgemeinen pro Mol Acetal der Formel II 5 bis 20 Mol, vorzugsweise 12 bis 16 Mol Methanol; 1 bis 3,5 Mol, vorzugsweise etwa 2 bis 2,5 Mol an Nitrosylchlorid bzw. Alkylnitrit und etwa 0,5 bis 3,0, vorzugsweise 1,0 bis 2,0 Mol Chlorwasserstoff.

Die Umsetzung wird im allgemeinen ohne Verwendung weiterer Lösungsmittel durchgeführt, da das Methanol als Lösungsmittel dient. Sofern Lösungsmittel mitverwendet werden, wählt man zweckmäßig solche, die gegenüber dem Nitrosierungsmittel und gegenüber H Cl inert sind, beispielsweise aliphatische, cycloaliphatische oder aromatische Kohlenwasserstoffe oder Halogenkohlenwasserstoffe.

Die Umsetzung wird im allgemeinen bei Temperaturen von —10°C bis 70°C, vorzugsweise 0 bis 25°C durchgeführt. Die Reaktionszeit liegt im allgemeinen zwischen etwa 30 Minuten und 2 Stunden. Im allgemeinen arbeitet man drucklos, doch läßt sich die Reaktion auch mit Vorteil bei erhöhtem Druck, beispielsweise einem Druck von bis zu 10 atü durchführen.

Die Umsetzung kann sowohl diskontinuierlich, als auch kontinuierlich durchgeführt werden. Bei der diskontinuierlichen Arbeitsweise trägt man zweckmäßig das Nitrosierungsmittel und eine methanolische HCl-Lösung in eine methanolische Lösung der Ausgangsverbindung II im Maße der Umsetzung ein. Bei der kontiuierlichen Arbeitsweise bringt man beispielsweise in ein mit einem Überlauf versehenes Reaktionsgefäß kontinuierlich die Reaktionspartner und den Katalysator ein.

Die Aufarbeitung des Reaktionsgemisches kann prinzipiell in üblicher Weise durch Destillation erfolgen. Zweckmäßig wird hierbei vor der Destillation der Chlorwasserstoff mit einer Base neutralisiert. Das gebildete Acetal der Formel Ia läßt sich auch nach Entfernung des Lösungsmittels beispielsweise durch Extraktion des mit Wasser versetzten Reaktionsgemisches mit einem mit Wasser nicht mischbaren Lösungsmittel, beispielsweise Benzol oder Methylenchlorid, und anschließende Destillation der organischen Phase isolieren. Zur Herstellung der 3 - Methyl - 2 - buten - 1,4 - dial - bisacetale bzw. zur Herstellung der 3 - Methyl - fumaraldehyd - 1 - monoacetale empfiehlt es sich jedoch das bei der Umsetzung A erhaltene Reaktionsgemsich ohne Abtrennung von nichtumgesetztem II direkt weiterzuverarbeiten und erst dann die Abtrennung von unumgesetztem Ausgangsmaterial sowie von gebildeten Nebenprodukten vorzunehmen.

Zur Eliminisierung von H Cl aus den Verbindungen Ia unter Bildung der entsprechenden Verbindungen Ib verwendet man mit Vorteil starke Basen wie NaOH, KOH, Natriumalkanolate oder Kaliumalkanolate in einem Lösungsmittel. Zweckmäßig verwendet man die Basen in alkanolischer Lösung. So eignet sich insbesondere die Lösung von NaOH, KOH, $NaOCH_3$, $KOCH_3$, $NaOC_2H_5$, $KOC_2H_5$, K-tert-$C_4H_9$ oder Na-tert-$C_4H_9$ in den entsprechenden Alkanolen.

Die starken Basen verwendet man im allgemeinen in Mengen von 1,5 Mol pro Mol Ia; die Alkohole in Mengen von etwa 5 bis 10 Mol pro Mol Ia. Die Reaktionszeit beträgt im allgemeinen 30 Minuten bis 6 Stunden vorzugsweise 60 bis 120 Minuten; die Reaktionstemperatur liegt im allgemeinen je

nach Art der starken Base und des Lösungsmittels zwischen 0 und 100°C, vorzugsweise 50 und 100°C.

Die Aufarbeitung des Reaktionsgemisches erfolgt auf übliche Weise durch Destillation. Die Ausbeute an dem gewünschten Produkt Ib betragen überraschender Weise etwa 80% der Theorie.

Bezüglich weiterer Einzelheiten über die Abspaltung von Halogenwasserstoff aus Alkylhalogeniden verweisen wir auf "Methoden der Organischen Chemie" Houben-Weyl Band 5/1B Seite 134 f.

Durch saure Hydrolyse können aus den Butendialbisacetalen der allgemeinen Formel Ib leicht die begehrten 3 - Methyl - fumardialdehyd - 1 - acetale hergestellt werden.

Die saure Hydrolyse erfolgt auf an sich bekannte Weise durch Behandlung mit einer wäßrigen Mineralsäure, wie Schwefelsäure oder Salzsäure, oder einer organischen Säure, wie Ameisensäure, p-Toluolsulfonsäure oder Essigsäure in Form einer 1 bis 20 %igen Lösung unter intensiver Vermischung. Die Reaktionsdauer beträgt im allgemeinen 0,5 bis 5, vorzugsweise 2 bis 3 Stunden, die Reaktionstemperatur etwa 10 bis 50°C.

Bei der Hydrolyse empfiehlt sich der Zusatz eines Lösungsvermittlers zum Reaktionsgemisch. Als Lösungsvermittler sind niedere aliphatische Alkohole, wie Methanol, Äthanol und Propanol sowie cycloaliphatische Äther, wie Tetrahydrofuran und Dioxan besonders geeignet. Die erhaltenen Aldehyde können auf übliche Weise, beispielsweise durch Extraktion nach schonender Neutralisation des Reaktionsgemisches, z.B. mit einem Alkalibicarbonat oder Natriumcarbonat, und Abdestillieren des Extraktionsmittels isoliert werden.

Während bei dieser sauren Hydrolyse die zur Methylgruppe $\alpha$-ständige Dimethylacetalgruppe rasch hydrolysiert, bleibt die Sechsringacetalgruppe wie bei der sauren Behandlung der Acetale der Formel II im Verfahrensschritt A überraschenderweise unangegriffen. Im Gegensatz hierzu ist die Sechsringacetalgruppierung in anderen ungesättigten Aldehyden auch leicht zu hydrolysieren. Setzt man z.B. ein erfindungsgemäß erhaltenes 3 - Methyl - fumardialdehyd - 1 - acetal in einer Wittigreaktion mit dem Ylid eines $\beta$ - Jonylidenäthyltriphenylphosphoniumsalzes um, so erhält man das Sechsringacetal von Retinal, dessen Acetalgruppierung unter den oben angegebenen Hydrolysebedingungen leicht gespalten werden kann. Hierdurch eröffnen sich viele Möglichkeiten durch successive Wittig-Reaktionen mit den 3 - Methyl - fumardialdehyd - 1 - monoacetalen zu verschiedenen Verbindungen mit biologischer und pharmakologischer Bedeutung zu gelangen.

Mit den 3 - Chlor - 3 - methyl - butan - 1,4 - dial - bisacetalen bzw. 3 - Methyl - 2 - buten - 1,4 - dial - bisacetalen wurden neue Verbindungen gefunden, über die die für Terpensynthesen begehrten 3 - Methyl - fumardialdehyd - 1 - monoacetale technisch besonders vorteilhaft erhalten werden können.

Beispiel 1

A Herstellung von 3 - Methyl - 3 - chlor - butan - 1,4 - dial - 4 - dimethylacetal - 1 - [2′,2′ - dimethyl - propylen]acetal. 17 g (0,1 Mol) 3 - Methyl - 3 - buten - 1 - al - [2′,2′ - dimethyl - propylen] - acetal wurden zusammen mit 15 ml Methylalkohol auf 0°C gekühlt. Zu dieser Lösung wurden im Laufe von 4 Stunden bei 0°C gleichzeitig 16 ml Isopropylnitrit und eine Lösung von 9 g HCl in 24 ml Methylalkohol zugetropft. Nach beendeter Zugabe wurde nach 10 Minuten bei dieser Temperatur gerührt und dann das Reaktionsgemisch durch Zugabe von 30%igem Natriummethanolat neutralisiert. Anschließend wurde filtriert und unter vermindertem Druck eingeengt. Das erhaltene Rohprodukt wurde mittels Gaschromatographie untersucht. Es enthielt 40,5% 3 - Methyl - 3 - chlor - butan - 1,4 - dial - 4 - dimethylacetal - 1 - [2′,2′ - dimethyl - propylen] - acetal neben 46,5% unumgesetzten Aufgangsmaterials und 13% Verunreinigungen. Die Ausbeute bezogen auf umgesetztes Ausgangsprodukt betrug 75,7%. Das Rohmaterial kann entweder destilliert oder zur weiteren Umsetzung direkt weiterverwendet werden. Bei der Destillation eines solchen Reaktionsansatzes erzielt man reines Verfahrensprodukt vom Siedepunkt 98—100°C bei 0,2 mbar und folgenden NMR-spektroskopischen Daten: $^1$H—NMR (Lösungsmittel $CDCl_3$; $\delta$ in ppm; TMS als inmerer Standart) $\delta = 4,75$ (t, 1H); 4,20 (S, 1H); 3,5 (m4H + 6H); 2,15 (d, 2H); 1,5 (s, 3H); 1,15 (s, 3H); 0,70 (s, 3H).

B Herstellung von 3 - Methyl - 2 - buten - 1,4 - dial - 4 - dimethyl - acetal - 1 - [2′,2′ - dimethyl - propylen] - acetal. Das gemäß Beispiel 1A erhaltene Rohmaterial wurde mit 100 ml einer 30%igen Natriummethanolat - Lösung versetzt und 3 Stunden unter Rückfluß zum Sieden erhitzt. Das erhaltene Reaktionsgemisch wurde mittels Gaschromatographie untersucht. Es ergab sich folgendes Bild: von dem gemäß 1A gebildeten 3 - Methyl - 3 - chlor - butan - 1,4 - dial - 4 - dimethylacetal - 1 - [2′,2′ - dimethyl - propylen] - acetal waren 88% in das gewünschte Olefin überführt und der Rest in Doppelbindungsisomere und Nebenprodukte. Das entspricht einer Ausbeute von 88%, bezogen auf eingesetztes Chlorbutan. Die Destillation dieses Reaktionsgemisches ergab 10 g 3 - Methyl - 2 - buten - 1,4 - dial - 4 - dimethylacetal - 1 - [2′,2′ - dimethyl - propylen] - acetal vom Siedepunkt 84°C bei 0,1 mbar

C Herstellung von 3 - Methyl - 2 - buten - 1,4 - dial - 1 - [2′,2′ - dimethyl - propylen] - monoacetal. 10 g des gemäß Beispiel 1B erhaltenen Bisacetals wurden in 50 ml

Methylenchlorid gelöst und bei Raumtemperatur mit 50 ml einer 3%igen Salzsäure gerührt. Nach 2 Stunden zeigte die gaschromatographische Analyse, daß sich alles Ausgangsmaterial umgesetzt hatte und quantitativ in das 3 - Methyl - fumardialdehyd - 1 - monoacetal übergegangen war.

Beispiel 2

A 17 g (0,1 Mol) 3 - Methyl - 3 - buten - 1 - al - [2',2' - dimethyl - propylen] - acetal wurden in 20 ml Methylalkohol gelöst. Zu dieser Lösung wurden bei 0°C im Verlauf von 1 Stunde gleichzeitig die Lösungen von 12,2 g (0,2 Mol) Methylnitrit in 20 ml Methylalkohol und von 10 g HCl in 10 ml Methylalkohol zugegeben. Nach beendeter Zugabe wurde noch 30 Minuten bei 0°C gerührt und dann mit 30% igem Natriummethanolat in Methanol neutralisiert. Durch gaschromatographische Analyse des Reaktionsgemisches bestimmt man eine Ausbeute von 78% der Theorie an 3 - Methyl - 3 - chlor - butan - 1,4 - dial - 4 - dimethylacetal - 1 - [2',2' - dimethyl - propylen] - acetal bei einem Umsatz von 71%. Destillative Aufarbeitung des Reaktionsgemisches ergab ein reines Produkt vom Siedepunkt Kp = 92°C bei 0,1 mbar und folgenden NMR-spektroskopischen Daten. $^1H$—NMR (Lösungsmittel $CDCl_3$; $\delta$ in ppm; TMS als innerer Standard); $\delta = 4{,}75$ (t, 1H); 4,20 (s, 1H), 3,3—3,6 (m, 6H); 2,1 (d, 2H); 1,5 (s, 3H); 1,15 (s, 3H); 0,70 (s, 3H).

B Eliminierung von HCl mittels Natrium-tert-butanolat 10 g eines gemäß Beispiel 2A erhaltenen reinen Chlorbutans wurden in 100 ml tert-Butanol gelöst, hierzu 3 g pulverisiertes Natriumhydroxid addiert und das Reaktionsgemisch 4 Stunden unter Rühren und unter Rückfluß zum Sieden erhitzt. Nach dem Abkühlen wurde das Reaktionsgemisch mit Essigsäure neutralisiert, der gebildete Niederschlag abfiltriert und das Filtrat unter vermindertem Druck eingeengt. Man erhielt 9,1 g eines öligen Rückstandes, der nach gaschromatographischer Analyse zu 81,4% aus dem gewünschten 3 - Methyl - 2 - buten - 1,4 - dial - 4 - dimethylacetal - 1 - [2',2' - dimethyl - propylen] - acetal bestand. Durch anschließende Destillation unter stark vermindertem Druck erhielt man 6,24 g des gewünschten Butendialbisacetals vom Siedepunkt Kp = 78°C bei 0,1 mbar, das gemaß GC analyse eine Reinheit von 96% aufwies.

**Patentansprüche**

1. Chlorbutan- bzw. Butendialbisacetale der allgemeinen Formel I

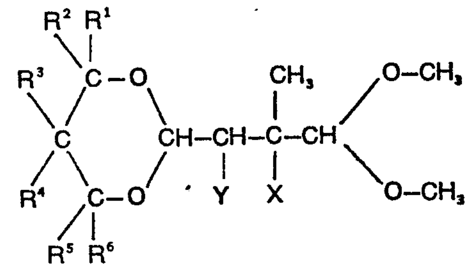

(I),

in der
$R^1$ bis $R^6$ für H, —$CH_3$ oder —$C_2H_5$, vorzugsweise H oder —$CH_3$ stehen, wobei vorzugsweise nur 1 bis 4 der Reste $R^1$ bis $R^6$ für —$CH_3$ und die restlichen für —H stehen;
und
Y für H steht wenn X Cl bedeutet (Ia)
oder
Y und X zusammen für eine weitere Bindung zwischen den Y und X tragenden C-Atomen stehen (Ib)

2. Verfahren zur Herstellung der Chlorbutan- bzw. Butendialbisacetale der allgemeinen Formel I

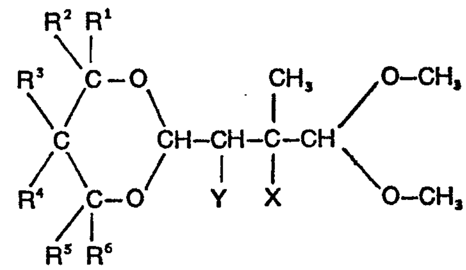

(I),

in der
$R^1$ bis $R^6$ für H, —$CH_3$ oder —$C_2H_5$, vorzugsweise H oder —$CH_3$ stehen, wobei vorzzugsweise nur 1 bis 4 der Reste $R^1$ bis $R^6$ für —$CH_3$ und die restlichen für —H stehen;
und
Y für H steht wenn X Cl bedeutet
oder aber
Y und X zusammen für eine weitere Bindung zwischen den Y und X tragenden C-Atomen stehen, dadurch gekennzeichnet, daß man

A Ein Sechsringacetal von 3 - Methyl - 3 - buten - 1 - al der allgemeinen Formel II

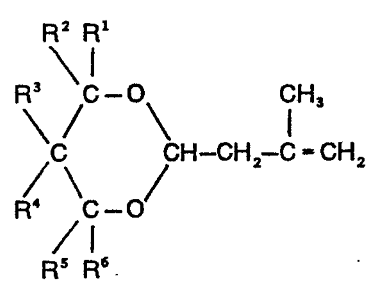

(II),

in Gegenwart von Methanol, Chlorwasserstoff und gegebenenfalls einem unter den Reaktionsbedingungen inerten Lösungsmittel mit Nitrosylchlorid oder einem Salpetrigsäureester der allgemeinen Formel III

$$R^7—O—N=O \qquad (III),$$

in der

$R^7$ für Alkyl mit 1 bis 6 C-Atomen, insbesondere Methyl, Äthyl, Isopropyl oder Cyclohexyl steht zu Verbindungen der Formel I, in der Y für H und X für Cl steht (Ia) umsetzt und

B gewünschtenfalls aus diesen in an sich bekannter Weise H Cl eliminiert.

3. Verwendung der neuen Butendialbisacetale der allgemeinen Formel I zur Herstellung von 3 - Methyl - fumardialdehyd - 1 - monoacetalen der allgemeinen Formel IV

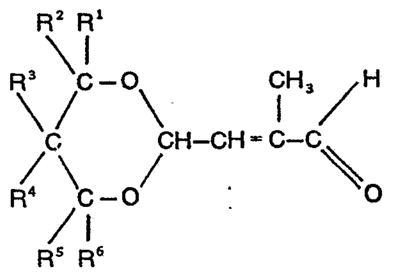

(IV),

in der $R^1$ bis $R^6$ für H, $—CH_3$ oder $—C_2H_5$ vorzugsweise H oder Methyl stehen; wobei vorzugsweise nur 1 bis 4 der Reste $R^1$ bis $R^6$ für $—CH_3$ und die restlichen für H stehen, dadurch gekennzeichnet, daß man die neuen Butendialbisacetale der allgemeinen Formel Ib

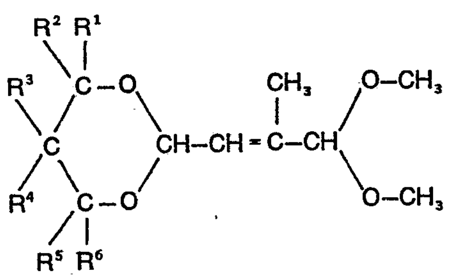

(Ib)

durch Einwirken verdünnter wäßriger Säuren verseift.

4. Verfahren zur Herstellung von 3 - Methyl - fumardialdehyd - 1 - monoacetalen der allgemeinen Formel IV

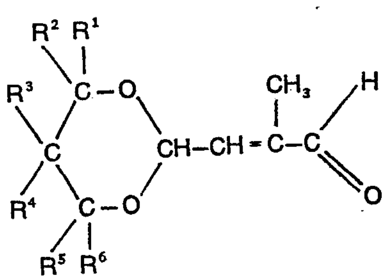

(IV),

in der $R^1$ bis $R^6$ die oben angegebene Bedeutung haben, dadurch gekennzeichnet, daß man

A ein Sechsringacetal von 3 - Methyl - 3 - buten - 1 - al der allgemeinen Formel II

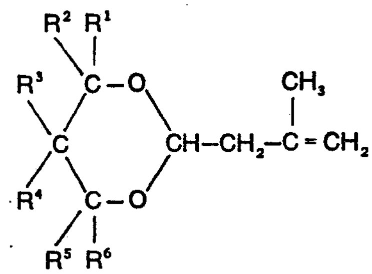

(II),

in Gegenwart von Methanol, Chlorwasserstoff und gegebenenfalls einem unter den Reaktionsbedingungen inerten Lösungsmittel mit Nitrosylchlorid oder einem Salpetrigsäureester der allgemeinen Formel III

$$R^7—O—N=O \qquad (III),$$

in der

$R^7$ für Alkyl mit 1 bis 6 C-Atomen, insbesondere Methyl, Äthyl oder Isopropyl steht, umsetzt;

B aus den so erhaltenen neuen Chlorbutandialbisacetalen der allgemeinen Formel Ia

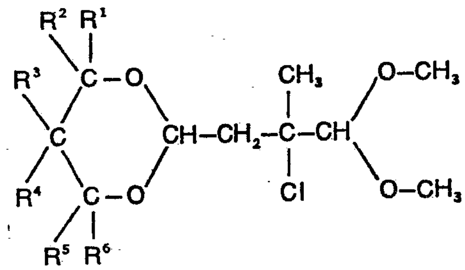

(Ia),

in der $R^1$ bis $R^6$ die oben angegebene Bedeutung haben, Chlorwasserstoff eliminiert, und

C die dabei erhaltenen 3 - Methyl - fumardialdehydbisacetale) der allgemeinen Formel Ib

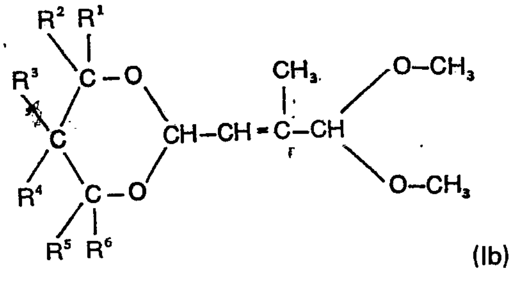

(Ib)

durch Einwirken verdünnter wäßriger Säuren verseift.

**Revendications**

1. Bis-acétals de chlorobutane- et butènedials de formule générale I

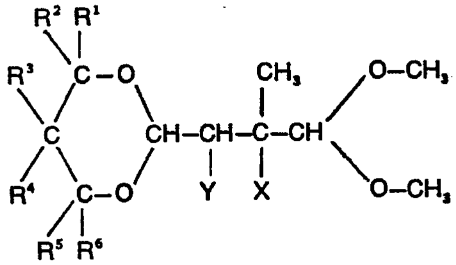

(I),

dans laquelle les symboles $R^1$ à $R^6$ représentent H, $—CH_3$ ou $—C_2H_5$, de préférence H ou $—CH_3$, et de préférence 1 à 4 seulement des symboles $R^1$ à $R^6$ représentent $—CH_3$ et les autres H; et Y représente H lorsque X représente Cl (Ia) ou bien Y et X forment ensemble une autre liaison entre les atomes de carbone portant les substituants Y et X (Ib).

2. Procédé de préparation des bis-acétals de chlorobutane- et butène-dials de formule générale I

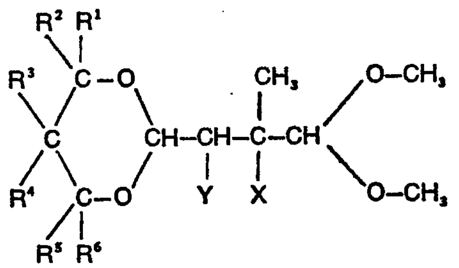

(I),

dans laquelle les symboles $R^1$ à $R^6$ représentent H, $—CH_3$ ou $C_2H_5$, de préférence H ou $—CH_3$, et de préférence 1 à 4 seulement des symboles $R^1$ à $R^6$ représentent $—CH_3$ et les autres H; et Y représente H lorsque X représente Cl ou bien Y et X forment ensemble une autre liaison entre les atomes de carbone portant les substituants Y et X, caractérisé en ce que

A) on fait réagir un acétal à cycle hexagonal du 3 - méthyl - 3 - butène - 1 - al, de formule générale II

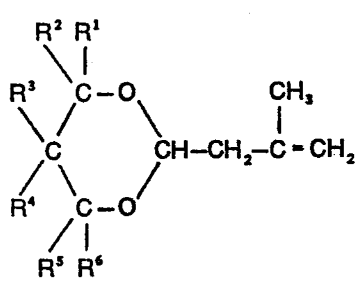

(II),

en présence de méthanol, de chlorure d'hydrogène et le cas échéant d'un solvant inerte dans les conditions de la réaction, avec du chlorure de nitrosyle ou un ester nitreux de formule générale III

$$R^7—O—N=O \qquad (III),$$

dans laquelle $R^7$ représente un groupe alkyle en C1—C6, plus spécialement un groupe méthyle, éthyle, isopropyle ou cyclohexyle, la reaction donnant des composés de formule I dans laquelle Y représente H et X représente Cl (Ia) et

B) si on le désire, on élimine HCl, de manière connue en soi, de ces composés.

3. Utilisation des nouveaux bis-acétals de butène-dial de formule générale I pour la préparation des 1-monoacétals du dialdéhyde 3 - méthyl - fumarique de formule générale IV

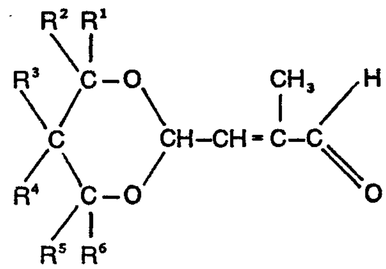

(IV),

dans laquelle les symboles $R^1$ à $R^6$ représentent H, $—CH_3$ ou $C_2H_5$, de préférence H ou un groupe méthyle; de préférence 1 à 4 seulement des symboles $R^1$ à $R^6$ représentent $—CH_3$ et les autres H, le procédé se caractérisant en ce que l'on soumet les nouveaux bis-acétals de butène-dial de formule générale Ib

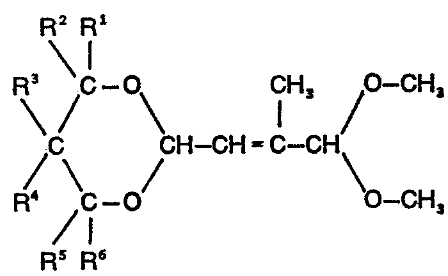

(Ib)

à saponification par action d'acides aqueux dilués.

4. Procédé de préparation des 1-monoacétals du dialdéhyde 3 - méthyl - fumarique de formule générale IV

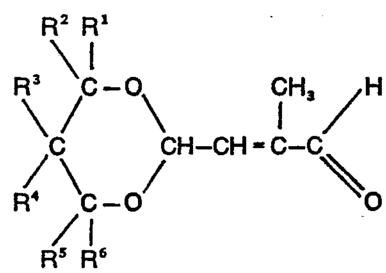

(IV),

dans laquelle les symboles $R^1$ à $R^6$ ont les significations indiquées ci-dessus, caractérisé en ce que

A) on fait réagir un acétal à cycle hexagonal du 3 - méthyl - 3 - butène - 1 - al de formule générale II

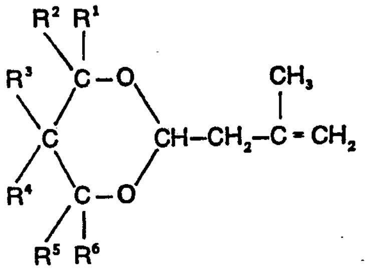

(II),

en présence de méthanol, de chlorure d'hydrogène et le cas échéant d'un solvant inerte dans les conditions de la réaction, avec du chlorure de nitrosyle ou un ester nitreux de formule générale III

$$R^7—O—N=O \quad (III),$$

dans laquelle $R^7$ représente un groupe alkyle en C1—C6, plus particulièrement un groupe méthyle, éthyle ou isopropyle;

B) dans les nouveaux bis-acétals de chlorobutane-dial ainsi obtenus répondant à la formule générale Ia

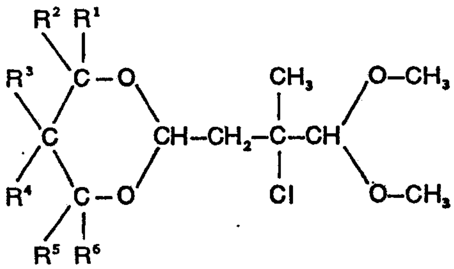

(Ia),

dans laquelle les symboles $R^1$ à $R^6$ ont les significations indiquées ci-dessus, on élimine le chlorure d'hydrogène, et

C) on soumet les bis-acétals du dialdéhyde 3 - méthyl - fumarique ainsi obtenus, répondant à la formule générale Ib

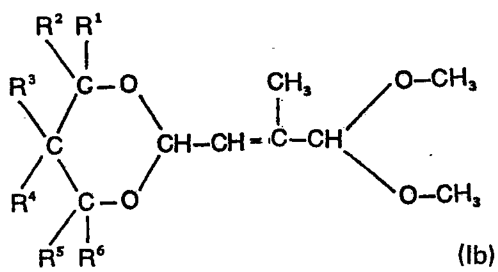

(Ib)

à saponification sous l'action d'acides aqueux dilués.

## Claims

1. Chlorobutanedial-bis-acetals and butenedial-bis-acetals of the general formula I

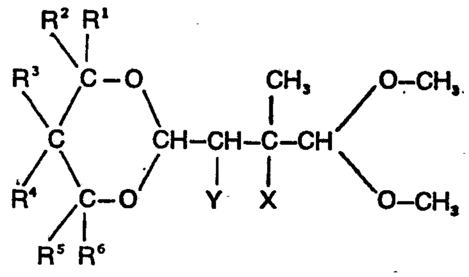

(I).

where $R^1$ to $R^6$ are H, $—CH_3$ or $—C_2H_5$, preferably H or $—CH_3$, but preferably only from 1 to 4 of the radicals $R^1$ to $R^6$ are $—CH_3$ and the remainder are —H, and Y is H if X is Cl (Ia) or Y and X together are a further bond between the carbon atoms bearing Y and X (Ib).

2. A process for the preparation of the chlorobutanedial-bis-acetals and butenedial-bis-acetals of the general formula I

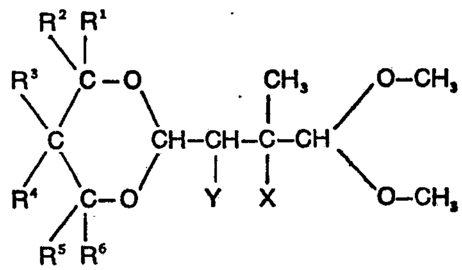

(I)

where $R^1$ to $R^6$ are H, $—CH_3$ or $—C_2H_5$, preferably H or $—CH_3$, but preferably only from 1 to 4 of the radicals $R^1$ to $R^6$ are $—CH_3$ and the remainder are —H, and Y is H if X is Cl or Y and X together are a further bond between the carbon atoms bearing Y and X, characterized in that

A. a six-membered cyclic acetal of 3 - methyl - but - 3 - en - 1 - al, of the general formula II

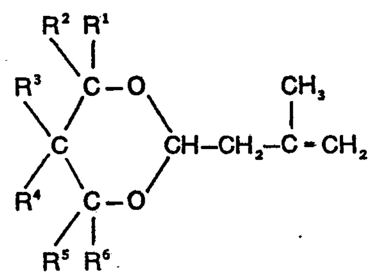

(II)

is reacted, in the presence of methanol and hydrogen chloride, and in the presence or absence of a solvent which is inert under the reaction conditions, with nitrosyl chloride or a nitrite ester of the general formula III

$$R^7—O—N=O \quad (III)$$

where $R^7$ is alkyl of 1 to 6 carbon atoms, especially methyl, ethyl, isopropyl or cyclohexyl, to give a compound of the formula I, where Y is —H and X is Cl (Ia) and,

B. if desired, HCl is eliminated from this

compound by a conventional method.

3. Use of the new butenedial-bis-acetals of the general formula I for the preparation of 3 - methyl - fumarodialdehyde - 1 - mono-acetals of the general formula IV

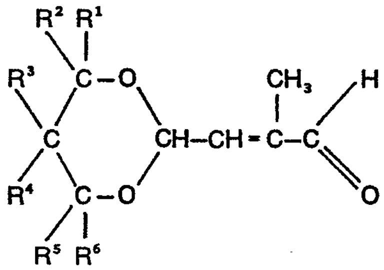

(IV)

where $R^1$ to $R^6$ are H, $—CH_3$ or $—C_2H_5$, preferably H or methyl, and preferably only from 1 to 4 of the radicals $R^1$ to $R^6$ are $—CH_3$ and the remainder are H, characterized in that the novel butenedial-bis-acetals of the general formula Ib

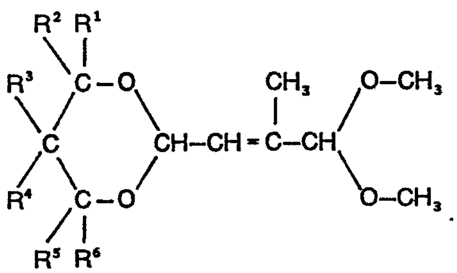

(Ib)

are hydrolyzed by treatment with a dilute aqueous acid.

4. A process for the preparation of 3 - methyl - fumarodialdehyde - 1 - mono-acetals of the general formula IV

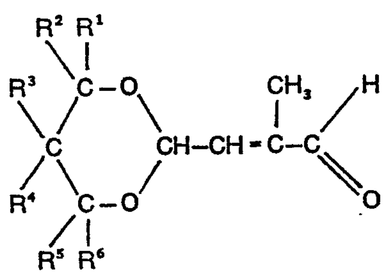

(IV)

where $R^1$ to $R^6$ have the above meanings, characterized in that

A. a six-membered cyclic acetal of 3 - methyl - but - 3 - en - 1 - al, of the general formula II

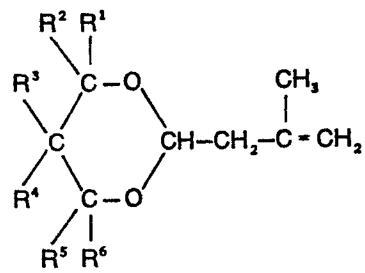

(II)

is reacted, in the presence of methanol and hydrogen chloride and in the presence or absence of a solvent which is inert under the reaction conditions, with nitrosyl chloride or a nitrite ester of the general formula III

$$R^7—O—N{=}O \qquad (III)$$

where $R^7$ is alkyl of 1 to 6 carbon atoms, especially methyl, ethyl or isopropyl,

B. if desired, hydrogen chloride is eliminated from the resulting novel chlorobutanedial-bis-acetals of the general formula Ia

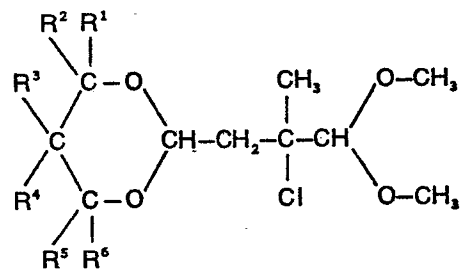

(Ia)

where $R^1$ to $R^6$ have the above meanings, and

C. the resulting methyl - fumarodialdehyde-bis-acetals of the general formula Ib

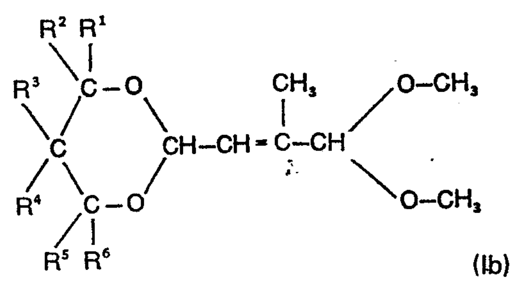

(Ib)

are hydrolyzed by treatment with a dilute aqueous acid.